(19) Europäisches Patentamt
European Patent Office
Office européen des brevets

(11) **EP 2 953 088 B1**

(12) **EUROPEAN PATENT SPECIFICATION**

(45) Date of publication and mention
of the grant of the patent:
**16.12.2020 Bulletin 2020/51**

(51) Int Cl.:
*G16H 20/10* *(2018.01)*    *G06Q 50/22* *(2018.01)*

(21) Application number: **14745407.8**

(86) International application number:
**PCT/JP2014/050898**

(22) Date of filing: **20.01.2014**

(87) International publication number:
**WO 2014/119401 (07.08.2014 Gazette 2014/32)**

(54) **INFORMATION PROCESSING DEVICE AND METHOD, AND PROGRAM**

INFORMATIONSVERARBEITUNGSVORRICHTUNG UND -VERFAHREN UND PROGRAMM

PROGRAMME, DISPOSITIF ET PROCÉDÉ DE TRAITEMENT D'INFORMATIONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.01.2013 JP 2013015520**

(43) Date of publication of application:
**09.12.2015 Bulletin 2015/50**

(73) Proprietor: **Sony Corporation
Tokyo 108-0075 (JP)**

(72) Inventors:
• **TAKAGI Yoshinori**
**Tokyo 108-0075 (JP)**
• **FUKUSHI Gakuho**
**Tokyo 108-0075 (JP)**
• **ARAYA Shinsuke**
**Tokyo 108-0075 (JP)**
• **HORIUCHI Yuya**
**Tokyo 108-0075 (JP)**

(74) Representative: **D Young & Co LLP
120 Holborn
London EC1N 2DY (GB)**

(56) References cited:
**EP-A1- 1 433 456      JP-A- 2002 132 940
JP-A- 2006 285 682      JP-A- 2012 113 523
US-A- 5 758 095         US-A1- 2006 149 480
US-B1- 7 072 840**

**Description**

Technical Field

The present technology relates to an information processing apparatus, an information processing method, and a program, and, in particular, to an information processing apparatus, an information processing method, and a program by which more accurate information on the dosage can be provided.

Background Art

Currently, prescriptions provided by doctors and medicine notebooks provided by pharmacies are handled as paper media. However, in order to improve the convenience and efficiency, it is desirable construct a system for electronically sharing and managing the prescriptions and the medicine notebooks.

In view of this, there has been proposed a technique for realizing safety medical prescription by managing prescription information pieces for patients in a medical prescription apparatus (e.g., see Patent Document 1). In this technique, when a user as a patient accesses the medical prescription apparatus, the medical prescription apparatus selects, from among the prescription information pieces for the patients, prescription information pieces satisfying a prescription condition instructed by a doctor and transmits them to a portable terminal of the patient.

The patient specifies desired one from among the prescription information pieces displayed on the portable terminal. This prescription information piece is presented to a dispensing pharmacy by the medical prescription apparatus. After that, the patient goes to the dispensing pharmacy. In the dispensing pharmacy, individual authentication or the like is performed. Based on the prescription information piece, a pharmacist in the dispensing pharmacy dispenses and delivers medicines to the patient.

Patent Document 1: Japanese Patent Application Laid-open No. 2004-029985

US 2006/149480 describes a method of making a medicament, and a medicament made thereby, including identifying at least one morbid condition including a plurality of manifestations for which a plurality of distinct active ingredients may be prescribed for treatment of each of the respective plurality of manifestations; determining a combination of at least two of the plurality of distinct active ingredients prescribed for treatment of each of the at least one condition, in which the combination may be taken together for such treatment of the respective plurality of manifestations; determining an effective dosage and content ratio of each of the distinct active ingredients in the combination; and preparing a medicament comprising the combination, wherein a ratio of each distinct active ingredient in the medicament substantially corresponds to the determined effective dosage and content ratio. The method similarly may be applied to co-morbid conditions.

US 5,758,095 A describes a system and method for ordering and prescribing drugs for a patient. This system includes an improved process for facilitating and automating the process of drug order entry. The user may interact with the system in a variety of ways such as keyboard, mouse, pen-base entry or voice entry. The system includes a database containing medical prescribing and drug information which is both general and patient-specific. The system also permits the user to view current and previously prescribed medications for any patient. The system can alert the user to potentially adverse situations as a result of the prescribed medication based on information in the database. The system also can automatically determine product selection based on descriptions and can automatically communicate the order to a pharmacy. Further, the system includes a means for automatically displaying messages to the user relating to predetermined situations. For example, such situations may include a medication which is not available in the formulary or the prescription of a non-recommended medication. The system streamlines the order entry process and makes information important to the drug ordering process easily available.

Summary of Invention

Problem to be solved by the Invention

By the way, if prescriptions and receipts are computerized, a prescription date and the number of prescription days for each prescription medicine prescribed for a patient can be extracted based on medication history information such as a receipt. Therefore, it is possible to know a dosage schedule of each prescription medicine. With the acquired dosage schedule, it is possible to avoid double administration of medicines and prescription of incompatible medicines to a user as a patient.

For example, it is assumed that Da and Ma are extracted from computerized receipts or the like as a prescription date and the number of prescription days for prescription medicines, respectively. These prescription medicines are taken by a patient in a period from the prescription date Da to Da+Ma-1. In this case, it is premised that the patient takes the prescription medicines everyday from the prescription date. Detection of incompatible medicines is carried out, for example, on the premise that the patient takes the prescription medicines from the period from Da to Da+Ma-1.

The number of prescription days for an oral medication or the like, that is, its dispensing quantity in a receipt is represented as the number of days, for example, "aa" days in which the medication is to be taken. On the other hand, this concept does not exist in some types of medicines such as a topical medication and a one-shot medicine. The dispensing quantity of these types of medicines is often described as "one."

This is because, for example, for the topical medication, a single tube can be prescribed as a medicine. In this case, the single tube contains a medicine for seven days. Even if the number of actually required prescription days is any one of one to seven days, only the single tube is prescribed as the medicine. Its dispensing quantity is likely to be described as "one." Thus, it is impossible to generate an accurate dosage schedule based on a prescription date and the number of prescription days described in a receipt or the like.

In addition, a prescription medicine such as a topical medication and a one-shot medicine, for which the number of prescription days represented as the dispensing quantity and the number of actually required dosing days are not identical to each other, is more than 1/3 of the total medicines that can be described in a receipt.

For some types of medicines such as a topical medication, the dispensing quantity of the prescription medicine, that is, the number of prescription days is not always identical to the number of dosing days in which a patient actually takes the medicines. It is sometimes impossible to obtain accurate information on the dosage.

The present technology has been made in view of the above-mentioned circumstances to obtain more accurate information on the dosage.

Means for solving the Problem

The invention is defined by the appended claims.

An information processing apparatus according to an aspect of the present technology includes: a determiner that identifies, based on information on pharmaceuticals prescribed for a user, dosage forms of the pharmaceuticals; and a dosage schedule generator that sets the pharmaceutical in a predetermined particular dosage form as a first processing-target pharmaceutical and determines, based on the dosage period for the first processing-target pharmaceutical, a dosage period for the pharmaceutical in another dosage form different from the particular dosage form, the pharmaceutical in the other dosage form having a dispensing date within a particular period including a dispensing date of the first processing-target pharmaceutical.

The dosage schedule generator may determine, based on the dispensing dates and the number of prescription days of the pharmaceuticals included in information on the pharmaceuticals, the dosage period for the first processing-target pharmaceutical.

The dosage schedule generator may set the number of days of the dosage period for the first processing-target pharmaceutical as the number of days of the dosage period for the pharmaceutical in the other dosage form.

The dosage schedule generator may set the longest dosage period among the dosage periods for the plurality of first processing-target pharmaceuticals, as the dosage period for the pharmaceutical in the other dosage form.

The particular dosage form may be an oral medication, an infusion, or a decoction.

The other dosage form may be an oral liquid medication, a one-shot medicine, an injection, a topical medication, or a material.

The dosage schedule generator may generate information indicating the dosage period for the pharmaceuticals and a dosage probability for the pharmaceuticals, as dosage schedule information.

The dosage schedule generator may calculate, based on the dosage period, the dosage probability for the pharmaceutical in a dosage form of a one-shot medicine such that the dosage probability on each date is gradually lowered according to the number of days from a prescription date.

The dosage schedule generator may calculate, if the dispensing date of the first processing-target pharmaceutical is not present in the particular period including the dispensing date of the pharmaceutical in the other dosage form, the dosage period for the pharmaceutical in the other dosage form by using an estimation equation calculated in advance by statistical processing based on the dosage period for the first processing-target pharmaceutical and a used amount of the pharmaceutical in the other dosage form.

An information processing method or a program according to an aspect of the present technology includes the steps of: identifying, based on information on pharmaceuticals prescribed for a user, dosage forms of the pharmaceuticals; and setting the pharmaceutical in a predetermined particular dosage form as a first processing-target pharmaceutical and determining, based on the dosage period for the first processing-target pharmaceutical, a dosage period for the pharmaceutical in another dosage form different from the particular dosage form, the pharmaceutical in the other dosage form having a dispensing date within a particular period including a dispensing date of the first processing-target pharmaceutical.

In an aspect of the present technology, based on information on pharmaceuticals prescribed for a user, dosage forms of the pharmaceuticals are identified. The pharmaceutical in a predetermined particular dosage form is set as a first processing-target pharmaceutical. Based on the dosage period for the first processing-target pharmaceutical, a dosage period for the pharmaceutical in another dosage form different from the particular dosage form is determined, the pharmaceutical in the other dosage form having a dispensing date within a particular period including a dispensing date of the first processing-target pharmaceutical.

Effect of the Invention

According to an aspect of the present technology, it is possible to obtain more accurate information on the dosage.

Brief Description of Drawings

[Fig. 1] A diagram showing a configuration example of an information processing system.
[Fig. 2] A diagram showing a configuration example of a data center.
[Fig. 3] A diagram explaining a prescription basic record and a dosage form code.
[Fig. 4] A diagram explaining a dispensing information record.
[Fig. 5] A flowchart explaining dosage schedule generation processing.
[Fig. 6] A diagram explaining a medical treatment identification code.
[Fig. 7] A flowchart explaining dosage schedule generation processing.
[Fig. 8] A diagram explaining dosage schedule information.
[Fig. 9] A diagram explaining a pharmaceutical record.
[Fig. 10] A diagram explaining an estimation equation calculation.
[Fig. 11] A diagram showing a configuration example of a data center.
[Fig. 12] A flowchart explaining dosage schedule generation processing.
[Fig. 13] A diagram showing a configuration example of a computer.

Mode(s) for Carrying Out the Invention

Hereinafter, referring to the drawings, embodiments to which the present technology is applied will be described.

<First Embodiment>

<Configuration Example of Information Processing System>

According to the present technology, for example, in the case where a prescription date and the number of prescription days of medicines prescribed to a patient are used to perform processing such as detecting double administration of medicines and incompatibility of medicines, accurate information on the dosage of the prescription medicines is obtained.
In the current state, information easily available as the prescription medicine information, i.e., medication history information is not a medication calendar showing a dosage schedule for a patient but data of a medical fee receipt such as a medical receipt and a pharmaceutical receipt. The medical receipt and the pharmaceutical receipt include a prescription date and the number of prescription days of prescription medicines. Note that the number of prescription days refers to days representing a prescribed quantity.
However, as mentioned above, for some types of medicines, the number of prescription days represented as the dispensing quantity is not identical to the number of actually required dosing days.
In view of this, in the present technology, it is an object to obtain more accurate information on the dosage of prescription medicines by complementing insufficient information on the prescription medicines in conjunction with other data on the prescription medicines such that a dosage schedule conforms to an application and the like utilizing a medication history. In other words, it is an object to provide a more accurate dosage schedule.
Next, a specific embodiment to which the present technology is applied will be described.

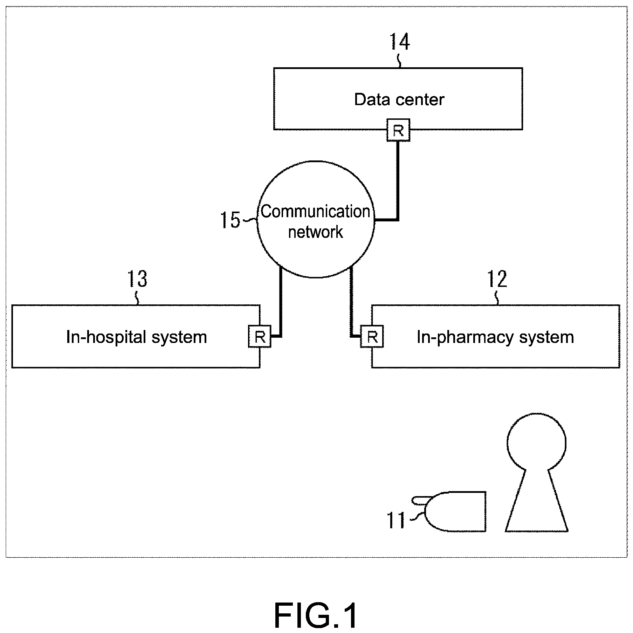

Fig. 1 is a diagram showing a configuration example according to an embodiment of an information processing system to which the present technology is applied.
The information processing system of Fig. 1 is constituted of a portable terminal apparatus 11, an in-pharmacy system 12, an in-hospital system 13, and a data center 14. The in-pharmacy system 12 to the data center 14 are connected to one another via a communication network 15 that is a wired or wireless network such as the Internet.
The portable terminal apparatus 11 is, for example, a cellular phone held by a user. The portable terminal apparatus 11 communicates with the in-pharmacy system 12 to the data center 14 via a communication network (not shown) or the communication network 15 and exchanges information if necessary.

The in-pharmacy system 12 is placed in a pharmacy from which the user purchases prescription medicines. The in-pharmacy system 12 is formed of one or more apparatuses. The in-pharmacy system 12 communicates with the portable terminal apparatus 11 to exchange necessary information with the portable terminal apparatus 11 or perform various types of processing according to an input operation of a pharmacist or the like.

Further, in the in-pharmacy system 12, personal information of a user as a patient, data on a medication history (hereinafter, referred to as medication history data), personal/medication history information including a dispensing pharmacy ID and the like for identifying the in-pharmacy system 12, pharmaceutical receipt information, and the like are recorded for each user.

The medication history data of each user includes information on medicines dispensed to the user in a pharmacy or the like, information on prescription of the medicines, a medication history ID for identifying the medication history data, and the like. More specifically, for example, the medication history data includes information indicating prescription medicines and information on a prescription date, the number of prescription days, a medication history ID, and the like of the medicines.

Further, the pharmaceutical receipt information is an computerized receipt. The pharmaceutical receipt information includes information on a name of a medicine prescribed for a user, a prescription date, a dispensing date, the number of prescription days, a receipt ID for identifying the pharmaceutical receipt information, and the like.

The in-pharmacy system 12 generates and records the personal/medication history information and the pharmaceutical receipt information and transmits the medication history data, the pharmaceutical receipt information, and the like included in the personal/medication history information to the data center 14 depending on needs.

The in-hospital system 13 is placed in a hospital to which a user as a patient goes. The in-hospital system 13 is formed of one or more apparatuses. The in-hospital system 13 communicates with the portable terminal apparatus 11 to exchange necessary information with the portable terminal apparatus 11 and perform various types of processing according to an input operation made by a doctor or the like.

Further, in the in-hospital system 13, personal information of the user as the patient, data on a medical examination (hereinafter, referred to as medical examination data), personal/medical examination information including a medical institution ID for identifying the in-hospital system 13 and the like, medical receipt information, DPC (Diagnosis Procedure Combination) receipt information, and the like are recorded for each user. For example, the medical examination data is an electronic health record.

Note that, for example, the medical receipt information or the DPC receipt information as the computerized receipt includes information on a name of medicine prescribed for a user as a patient, a prescription date and the number of prescription days of the medicine, a receipt ID for identifying the medical receipt information and the DPC receipt information, and the like.

The in-hospital system 13 generates and records the personal/medical examination information, the medical receipt information, and the DPC receipt information and transmits the medical examination data, the medical receipt information, and the DPC receipt information included in the personal/medical examination information to the data center 14 depending on needs.

The data center 14 is formed of one or more apparatuses. The data center 14 receives and records, from the in-pharmacy system 12 and the in-hospital system 13, the medication history data, the medical examination data, the pharmaceutical receipt information, the medical receipt information, the DPC receipt information, and the like. The data center 14 transmits these data pieces to the in-pharmacy system 12, the in-hospital system 13, and the portable terminal apparatus 11 depending on requirements. In other words, information on the medication history data, the medical examination data, and the like recorded in the data center 14 is shared with the in-pharmacy system 12, the in-hospital system 13, and the portable terminal apparatus 11.

<Configuration Example of Data Center>

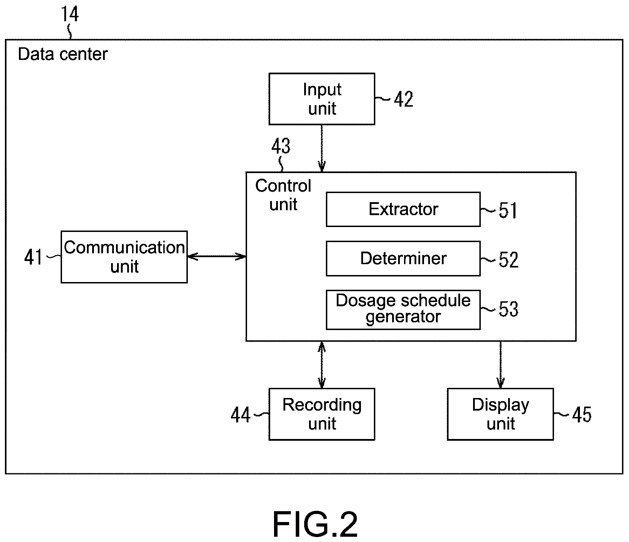

Next, a further detailed configuration of the data center 14 of Fig. 1 will be described. Fig. 2 is a diagram showing a further detailed configuration example of the data center 14.

The data center 14 is constituted of a communication unit 41, an input unit 42, a control unit 43, a recording unit 44, and a display unit 45.

The communication unit 41 communicates with an external apparatus such as the portable terminal apparatus 11 to receive various types of data and supply them to the control unit 43 and to transmit data supplied from the control unit 43. The input unit 42 is, for example, an input button or a touch panel. The input unit 42 supplies information input by a manager of the data center 14 to the control unit 43.

The control unit 43 controls a general operation of the data center 14. The control unit 43 includes an extractor 51, a determiner 52, and a dosage schedule generator 53.

The extractor 51 extracts, from information on prescription medicines prescribed for a patient, such as the pharmaceutical receipt information recorded in the recording unit 44, information on the dosage form code, the prescription date, the number of prescription days, and the like of the prescription medicines.

The determiner 52 determines, based on a dosage form code of each extracted prescription medicine, a dosage form of each prescription medicine. The dosage schedule generator 53 generates, according to the result of the determination of the determiner 52, dosage schedule information indicating a dosage schedule of each prescription medicine.

The recording unit 44 records a program and various types of data executed by the control unit 43 and supplies these data pieces to the control unit 43 depending on needs. For example, the recording unit 44 records the user medication history information, the pharmaceutical receipt information, the medical receipt information, the DPC receipt information, and the like. The display unit 45 is, for example, a liquid-crystal display panel. The display unit 45 displays various images based on the data supplied from the control unit 43.

<Explanation of Dosage Schedule Generation Processing>

Based on information on prescription medicines prescribed for a user, which is recorded in the recording unit 44, the data center 14 generates dosage schedule information of each prescription medicine. Hereinafter, it is assumed that the pharmaceutical receipt information is used as the information on the prescription medicines to generate the dosage schedule information.

For example, the pharmaceutical receipt information includes on information on one or more prescriptions. The information on each prescription further includes information on one or more prescription medicines.

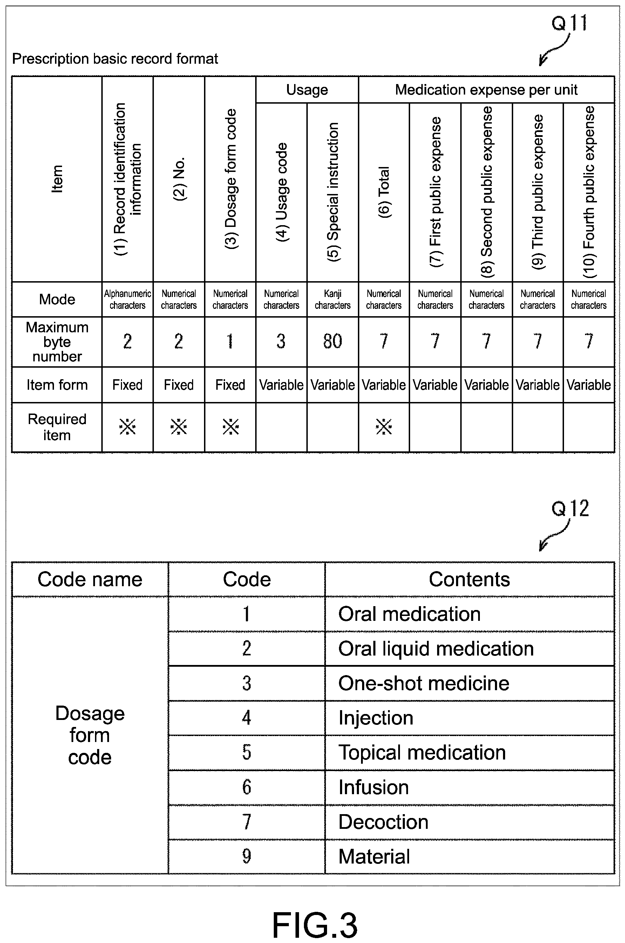

Specifically, the pharmaceutical receipt information includes a prescription basic record shown by the arrow Q11 of Fig. 3, for example, as the information on the prescription. The prescription basic record includes a dosage form code indicating a dosage form of a prescription medicine, a usage code indicating a dosing method for the prescription medicine, and the like.

Further, the dosage form code of the prescription medicine is defined for each dosage form as shown by the arrow Q12. Specifically, the dosage form code of an oral medication is set to "1." The dosage form code of an oral liquid medication is set to "2." The dosage form code of a one-shot medicine is set to "3." The dosage form code of an injection is set to "4." The dosage form code of a topical medication is set to "5." The dosage form code of an infusion is set to "6." The dosage form code of a decoction is set to "7." The dosage form code of a material is set to "9."

Based on the dosage form code of the prescription medicine extracted from the pharmaceutical receipt information as described above, it is possible to determine the dosage form of the prescription medicine.

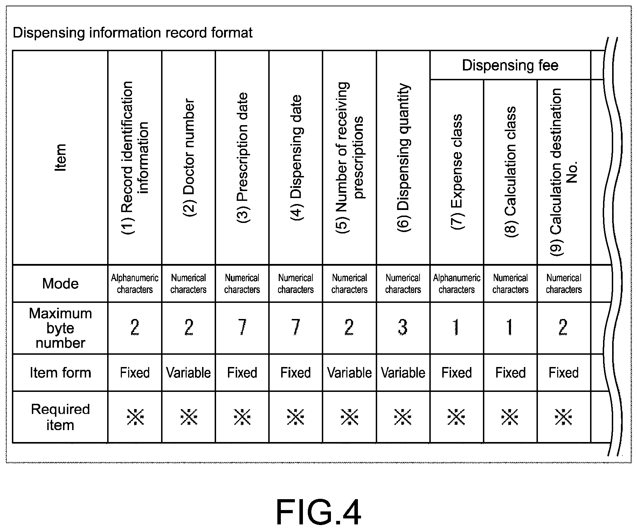

Further, the pharmaceutical receipt information includes a dispensing information record shown in Fig. 4, for example, as the information on the prescription medicines. The dispensing information record includes a prescription date, a dispensing date, a dispensing quantity, and the like of each prescription medicine. The dispensing quantity refers to a quantity of a prescribed prescription medicine. For example, if an oral medication for three days is prescribed, the number "3" indicating the number of prescription days is set.

Next, an operation of the data center 14 will be described.

For example, when the user operates the portable terminal apparatus 11 to start an application program associated with notification of the danger of the co-administration of medications, the portable terminal apparatus 11 requests the data center 14 to transmit dosage schedule information of each prescription medicine.

In response to the request of the portable terminal apparatus 11, the data center 14 performs dosage schedule generation processing to generate dosage schedule information. Then, the data center 14 generates information indicating a dosage period (dosage schedule) of each prescription medicine as the dosage schedule information and transmits it to the portable terminal apparatus 11.

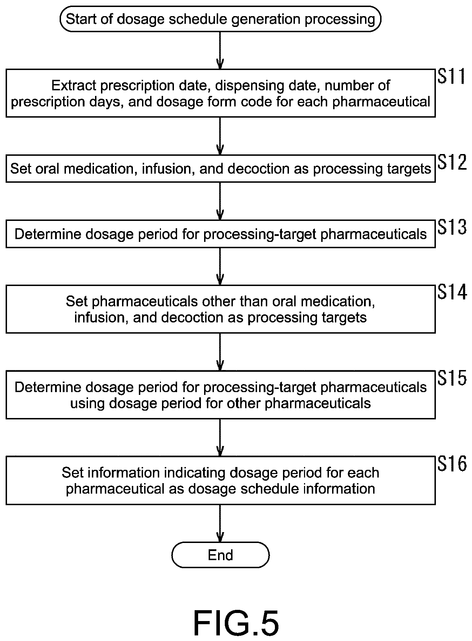

Hereinafter, referring to a flowchart of Fig. 5, the dosage schedule generation processing performed by the data center 14 will be described. Note that the dosage schedule generation processing is performed for each of the pharmaceutical receipt information pieces recorded in the recording unit 44, for example.

In Step S11, the extractor 51 reads out pharmaceutical receipt information from the recording unit 44 and performs analysis processing on the readout pharmaceutical receipt information, to thereby extract a prescription date, a dispensing date, the number of prescription days, and a dosage form code of each prescription for each pharmaceutical (prescription medicine) from the pharmaceutical receipt information. For example, the dispensing quantity shown in Fig. 4 is extracted as the number of prescription days.

In Step S12, the determiner 52 sets an oral medication, an infusion, and a decoction out of the pharmaceuticals (prescription medicines), the information of which has been extracted from the pharmaceutical receipt information, as processing-target pharmaceuticals. In other words, the pharmaceutical having a dosage form code of any of "1," "6," and "7," in other words, the oral medication, the infusion, and the decoction is set as the processing-target pharmaceutical.

In Step S13, the dosage schedule generator 53 determines, for each of the processing-target pharmaceuticals, based on the dispensing date and the number of prescription days of the pharmaceutical, a dosage period for each of the processing-target pharmaceuticals.

For example, if the dispensing date of a predetermined pharmaceutical is Da and the number of prescription days is Ma, the dosage schedule generator 53 sets a dosage start date to Da, a dosage end date to Da+Ma-1, and a period from Da and Da+Ma-1 as a dosage period (dosage schedule) of the pharmaceutical. Although the example in which the dispensing date is the dosage start date is described here, the prescription date may be the dosage start date. This is because the prescription date is often identical to the dispensing date.

Typically, the dispensing quantity of pharmaceuticals such as an oral medication, an infusion, and a decoction is represented by a value of the number of dosing days with days in which they are taken being a unit. Therefore, the dispensing quantity, i.e., the number of prescription days obtained from the pharmaceutical receipt information is in many cases identical to the number of actually required dosing days. Therefore, regarding pharmaceuticals such as an oral medication, an infusion, and a decoction, an almost accurate dosage schedule thereof can be obtained based on the dispensing date, the number of prescription days, and the like of these pharmaceuticals that are obtained from the pharmaceutical receipt information.

Although the example in which the number of prescription days of the pharmaceuticals is used as the number of dosing days as it is for determining the dosage period is described here, the dosage period for the processing-target pharmaceuticals may be calculated by using, for example, information on the dosage period for other pharmaceuticals and each prescription of the pharmaceuticals.

For example, the medicinal effects of some of the pharmaceuticals last for a few days after they are taken. Such a medicine will be referred to as a long-term effective medicine. It is assumed that the medicinal effect of the long-term effective medicine lasts for seven days, for example. In this case, for example, if the long-term effective medicine for two weeks is prescribed, then the number of prescription days is two days. Therefore, the dosage period determined based on the number of prescription days is not identical to the actually required dosage period.

Therefore, if the processing-target pharmaceutical is the long-term effective medicine, the dosage schedule generator 53 determines a dosage period, considering the number of prescription days, i.e., the number of dosing days of this pharmaceutical as being identical to the number of prescription days of the other processing-target pharmaceuticals. Whether or not the pharmaceutical is the long-term effective medicine can be determined by division using the number of prescription days of the pharmaceutical and the number of prescription days of the other processing-target pharmaceuticals.

Specifically, for example, it is assumed that a pharmaceutical A is a medicine to be taken everyday, a pharmaceutical B is a long-term effective medicine to be taken once a week, and the pharmaceutical A and the pharmaceutical B for two weeks are prescribed. In this case, the number of prescription days Ma equals 14 because the pharmaceutical A is taken everyday and the number of prescription days Mb equals 2 because the pharmaceutical B is taken once a week. Consequently, a remainder Z of Ma/Mb=14/2 is zero. When the remainder Z of Ma/Mb is zero, it is determined that the pharmaceutical B is the long-term effective medicine and the number of dosing days of the pharmaceutical B is Ma. Where Ma>Mb is established.

Although the case where the remainder Z is zero is described here as the example in which the pharmaceutical B is the long-term effective medicine, it is also possible to determine whether or not the pharmaceutical B is the long-term effective medicine by judging whether or not another condition is satisfied.

Further, some of the pharmaceuticals are gradually decreased or increased in prescription amount. Such pharmaceuticals are in some cases divided in a plurality of prescriptions and dispensed on the same dispensing date.

For example, those pharmaceuticals are divided in a prescription for first three days and a prescription for the next three days. Thus, the pharmaceuticals for total six days are prescribed. The pharmaceuticals of the prescriptions are dispensed on the same date. In this case, it is difficult to determine the number of actually required dosing days based on the number of prescription days and the prescription date (dispensing date) obtained from the pharmaceutical receipt information.

However, it is possible to estimate, based on a calculation section, a usage code, a remarks column, and the like included in the pharmaceutical receipt information, whether or not the same pharmaceutical divided in the plurality of prescriptions and dispensed on the same date is to be taken on the same date.

For example, if the calculation section is "3," it means the gradual dosage tapering schedule and the dispensing quantity is not totalized. It can be seen that the prescription amount of the pharmaceuticals is gradually decreased. Therefore, in such a case, the sum of the number of prescription days of the prescriptions of the pharmaceuticals only needs to be considered as the number of actually required dosing days. If dosing timings indicated by usage codes in different prescriptions overlap with each other, the pharmaceuticals related to these prescriptions should be taken on different dates. Therefore, the sum of the number of prescription days of the prescriptions only needs to be considered as the number of actually required dosing days.

If it is determined by the dosage schedule generator 53 that the same pharmaceutical divided in the plurality of prescriptions and dispensed on the same date is to be taken on different dates as described above, the sum of the number of prescription days of these prescriptions may be considered as the number of actually required dosing days.

In the above-mentioned manner, the dosage schedule generator 53 determines, for each of the processing-target pharmaceuticals, based on the dispensing date and the number of prescription days of the pharmaceutical, the dosage period for each processing-target pharmaceutical.

Hereinafter, the dosage period obtained by the processing of Step S13 will be also referred to as a dosage period Ta. In addition, the pharmaceuticals set as the processing targets in the processing of Step S12, i.e., the oral medication, the infusion, and the decoction will be also referred to as first processing-target pharmaceuticals.

In Step S14, the determiner 52 sets, pharmaceuticals other than the oral medication, the infusion, and the decoction out of the pharmaceuticals (prescription medicines) the information of which has been extracted from the pharmaceutical receipt information, as processing targets. Specifically, the pharmaceutical having a dosage form code of any of "2," "3," "4," "5," and "9," i.e., any one of the oral liquid medication, the one-shot medicine, the injection, the topical medication, and the material is set as the processing-target pharmaceutical.

In Step S15, the dosage schedule generator 53 determines, for each of the processing-target pharmaceuticals, using the dosage period Ta for the first processing-target pharmaceuticals set as the processing targets in the processing of Step S12, a dosage period for each processing-target pharmaceutical.

For example, the dosage schedule generator 53 sets the longest dosage period Ta among the dosage periods Ta for the first processing-target pharmaceuticals, as the dosage period for the processing-target pharmaceutical as it is.

Note that, if the dosage start date of the dosage period Ta is different from the dispensing date of the processing-target pharmaceutical, the dispensing date of the processing-target pharmaceutical may be set to the dosage start date and the dosage end date of the dosage period Ta as the dosage end date of the processing-target pharmaceutical. Alternatively, the dispensing date of the processing-target pharmaceutical may be set to the dosage start date, the number of days of the dosage period Ta may be set to the number of dosing days, and a period defined by the dosage start date and the number of dosing days may be set as the dosage period for the processing-target pharmaceutical.

For example, regarding the pharmaceutical such as a topical medication which is set as the processing target in the processing of Step S14, the dispensing quantity of the pharmaceutical is often set to "1" because it is handled as a certain dose, for example. That is, the number of prescription days represented by the dispensing quantity is often not identical to the number of actually required dosing days.

On the other hand, those pharmaceuticals are often prescribed in almost the same period as the other pharmaceuticals such as an oral medication. For example, an oral medication and a topical medication are in some cases prescribed in one medical examination. For example, there is also a case where an oral medication is prescribed in one medical examination, a patient takes the oral medication, experiences a symptom such as itching, and undergoes a medical examination again, and a topical medication is prescribed.

In this manner, the pharmaceutical such as a topical medication set as the processing target in the processing of Step S14 is prescribed in almost the same period as the pharmaceutical such as an oral medication set as the processing target in the processing of Step S12. In such a case, the actually required dosage period for the pharmaceutical such as a topical medication is mostly the same as the dosage period for the other pharmaceutical such as an oral medication prescribed in the same period.

Therefore, considering the dosage period for the pharmaceutical such as a topical medication set as the processing target in the processing of Step S14 as the dosage period Ta of the first processing-target pharmaceutical prescribed at almost the same time, the dosage schedule generator 53 overcomes the inconsistency between the number of prescription days and the number of dosing days which is caused with respect to the pharmaceutical such as a topical medication.

Note that, in the dosage schedule generation processing, the pharmaceuticals the information of which has been extracted from the pharmaceutical receipt information are set as the processing targets, and hence the other first processing-target pharmaceuticals prescribed in almost the same period as the pharmaceuticals set as the processing targets in Step S15 are pharmaceuticals in the same receipt as the processing-target pharmaceuticals.

Here, the pharmaceuticals prescribed in almost the same period are not limited to the pharmaceuticals in the same receipt. The pharmaceuticals prescribed in almost the same period only need to be pharmaceuticals whose prescription date (dispensing date) is within a particular period such as one month and one week. That is, the dosage period for the pharmaceutical set as the processing target in Step S15 only needs to be set to the dosage period for the first processing-target pharmaceuticals dispensed within the particular period including the dispensing date of this pharmaceutical.

Note that, if a plurality of dosage periods Ta having different dosage start dates are present in the particular period, the dosage period Ta for the pharmaceutical of the dosage start date, which is before the dispensing date of the processing-target pharmaceuticals and closest from the dispensing date of the processing-target pharmaceuticals among those dosage start dates, may be set as the dosage period for the processing-target pharmaceuticals.

Although the above-mentioned particular period may be any period, it is effective to set the particular period to one month or one week, for example, in addition to the period in the same receipt.

This is because the receipt is created for about one month and pharmaceuticals for one month or more are rarely prescribed in one prescription. This is also because, for example, in the case where an oral medication is prescribed in a first medical examination, a medical examination is performed again because another symptom is caused after taking the oral medication, and a topical medication is prescribed, this second medical examination is often performed within one week from the first medical examination.

In the above-mentioned manner, the dosage period (dosage schedule) for the processing-target pharmaceuticals is determined, and then the processing proceeds from Step S15 to Step S16.

In Step S16, the dosage schedule generator 53 sets information indicating the dosage period for each pharmaceutical determined in the processing of Steps S13 and S15 as the dosage schedule information. The dosage schedule generation processing is terminated.

The dosage schedule information generated by the dosage schedule generator 53 is transmitted to the portable terminal apparatus 11 by the communication unit 41. With this, in the portable terminal apparatus 11, the dosage schedule information received from the data center 14 can be utilized in various application programs.

For example, if the dosage schedule information obtained at the data center 14 is used for detecting the incompatibility of medications, it is possible to improve the detection accuracy in comparison with the prior art.

Specifically, for example, it is assumed that a predetermined oral medication and isoprenaline sulfate being a topical medication for 30 days are prescribed for the patient on 1 December and the dispensing quantity of the isoprenaline sulfate in the receipt is set to "1," in other words, the number of prescription days is set to one day. It is also assumed that dl-Methylephedrine hydrochloride that must not be used with the isoprenaline sulfate is prescribed for the patient on 13 December.

In this case, in the prior art, the dosage period for the isoprenaline sulfate has been only indicated as 1 December and it has been impossible to detect the incompatibility with the dl-Methylephedrine hydrochloride. In contrast, in the data center 14, the dosage period for the isoprenaline sulfate is set to the same period as the oral medication prescribed on the same date, that is, a period from 1 to 30 December, and hence it is possible to detect the co-administration with the dl-Methylephedrine hydrochloride and issue an alarm.

In the above-mentioned manner, the data center 14 determines the dosage period Ta of the first processing-target pharmaceutical in a predetermined dosage form and calculates the dosage period for the pharmaceutical in the other dosage form by using the dosage period Ta.

As described above, the dosage period for the first processing-target pharmaceutical whose inconsistency between the number of prescription days and the number of actually required dosing days is small is used to determine the dosage period for the other pharmaceutical prescribed in almost the same period. Thus, it is possible to obtain more accurate dosage schedule information. In other words, it is possible to obtain more accurate information on the dosage.

Regarding the topical medication, for example, it is estimated that about 30% of all prescriptions relates to the topical medication. However, what is described in the receipt as the dispensing quantity of the topical medication has been not the number of prescription days but the prescribed quantity. Therefore, the present technology is effective to obtain a more accurate dosage schedule with respect to, in particular, the topical medication.

According to the present technology, if the dosage start date and the number of dosing days of the pharmaceuticals, i.e., the dosage schedule can be obtained based on the dispensing date and the dispensing quantity, which is the purpose of the receipts, it is possible to estimate a dosing activity of the user (patient) itself. If this dosing activity can be visualized, in addition to warning of the incompatibility of medicines, which is the original purpose of the medicine notebooks, it becomes possible to detect double administration of medicines and excessive administration of medicines and offer a health care suggestion depending on a change in dosing condition, for example.

Further, it is also possible to utilize the determined dosage schedule as a parameter when performing adaptive processing such as sorting out, archiving, and compressing information in the cooperation between medical institutions and the home medical care, which will increase in importance, for example.

Although the case where the information on the pharmaceuticals is extracted from the pharmaceutical receipt information has been described above, the information on the pharmaceuticals may be extracted from information such as medical receipt information.

It should be noted that, in such a case, the information to be extracted is stored in a format different from that used in the pharmaceutical receipt information, and hence re-read processing is appropriately required.

For example, when information on a pharmaceutical is extracted from the medical receipt information, a medical treatment identification code is acquired as information on the dosage form of the pharmaceutical.

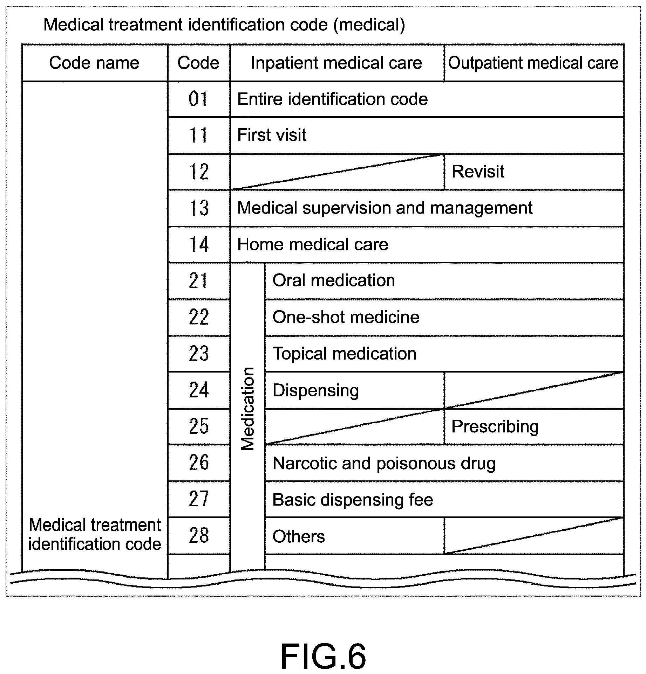

The medical treatment identification code includes a code indicating a dosage form of a dosed medicine as shown in Fig. 6. For example, if the dosed medicine is an oral medication, the medical treatment identification code is set to "21." If the medicine is a one-shot medicine, the medical treatment identification code is set to "22." Further, if the dosed medicine is a topical medication, the medical treatment identification code is set to "23."

The example in which the dosage schedule generation processing is performed at the data center 14 has been described above. Alternatively, the portable terminal apparatus 11 or another apparatus may perform the dosage schedule generation processing to generate the dosage schedule information of the pharmaceuticals.

<Second Embodiment>

<Explanation of Dosage Schedule Generation Processing>

Although the case where the dosage schedule information is the information indicating only the dosage schedule of each pharmaceutical has been described, the dosage schedule information may include not only the dosage schedule but also information indicating a dosage probability on each dosage date, that is, a probability for the patient (user) to take the pharmaceuticals.

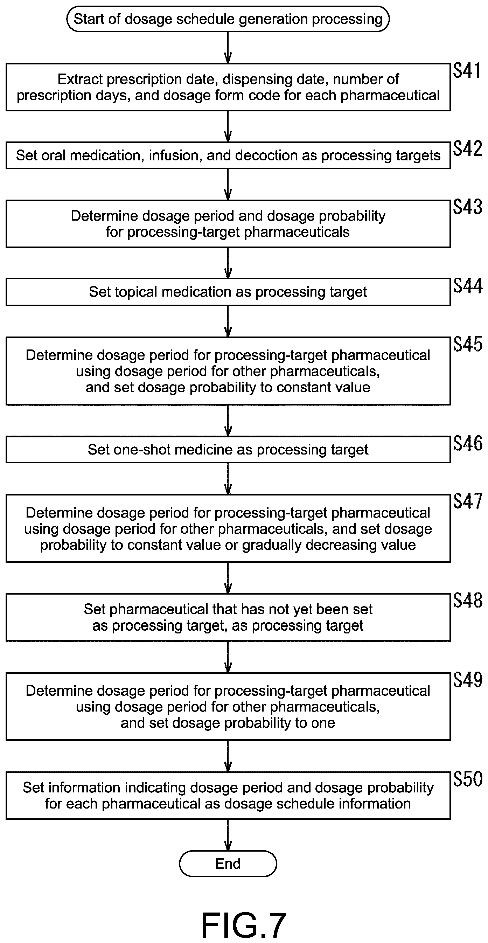

Hereinafter, referring to a flowchart of Fig. 7, dosage schedule generation processing performed by the data center 14 when generating dosage schedule information indicating a dosage schedule and a dosage probability will be described.

Note that the processing of Steps S41 and S42 are the same as the processing of Steps S11 and S12 of Fig. 5, and hence descriptions thereof will be omitted.

In Step S43, the dosage schedule generator 53 determines, for each of the processing-target pharmaceuticals, based on a dispensing date and the number of prescription days of the pharmaceutical, a dosage period and a dosage probability for each of the processing-target pharmaceuticals.

For example, the dosage schedule generator 53 performs the same processing as that of Step S13 of Fig. 5 to determine a dosage period Ta for each pharmaceutical. Then, the dosage schedule generator 53 sets, for example, with respect to each pharmaceutical, a dosage probability for the pharmaceutical in the dosage period Ta to "1" and a dosage probability in periods other than the dosage period Ta to "0."

In Step S44, the determiner 52 sets a topical medication out of pharmaceuticals (prescription medicines) the information of which has been extracted from the pharmaceutical receipt information, as a processing target. In other words, the pharmaceutical having a dosage form code of "5" is set as the processing-target pharmaceutical.

In Step S45, by using the dosage period Ta for the first processing-target pharmaceuticals, the dosage schedule generator 53 determines the dosage period for the processing-target pharmaceutical and sets the dosage probability for the pharmaceutical to a predetermined constant value.

For example, the dosage schedule generator 53 sets a longest dosage period Ta among the dosage periods Ta for the first processing-target pharmaceuticals, as the dosage period for the processing-target pharmaceutical as it is. Further, the dosage schedule generator 53 sets the dosage probability in the dosage periods Ta for the processing-target pharmaceutical to a constant K and the dosage probability in periods other than the dosage period Ta to "0" for providing a flat probability distribution.

Here, the constant K set as the dosage probability is a value of 0 or more and 1 or less. The constant K is defined for each pharmaceutical or each pharmacotherapeutic classification, for example. If information on the pharmacotherapeutic classification or the like of the pharmaceutical cannot be obtained, the constant K =1 may be set. By defining the value of the constant K for each pharmaceutical or each pharmacotherapeutic classification in this manner, it is possible to define a more suitable dosage probability for each pharmaceutical.

In Step S46, the determiner 52 sets a one-shot medicine out of the pharmaceuticals (prescription medicines) the information of which has been extracted from the pharmaceutical receipt information, as a processing target. In other words, a pharmaceutical having a dosage form code of "3" is set as the processing-target pharmaceutical.

In Step S47, by using the dosage period Ta for the first processing-target pharmaceuticals, the dosage schedule generator 53 determines the dosage period for the processing-target pharmaceutical and sets the dosage probability for the pharmaceutical to a predetermined constant value or a gradually decreasing value.

For example, the dosage schedule generator 53 sets the longest dosage period Ta among the dosage periods Ta for the first processing-target pharmaceuticals, as the dosage period for the processing-target pharmaceutical as it is.

Further, the dosage schedule generator 53 calculates the dosage probability in the dosage period Ta for the processing-target pharmaceutical by calculating, for example, Expression (1) below.

$$\text{Dosage probability} = K * N/A * F(x) \qquad \cdots (1)$$

Note that, in Expression (1), K is a dosage probability as a reference, and is, for example, a constant K indicating a value of a dosage probability for a topical medication. N indicates a prescription amount (number of prescription days) of the processing-target pharmaceutical. Further, A indicates the number of days of the dosage period Ta and F(x) indicates a gradually decreasing function with x being days from the prescription date of the processing-target pharmaceutical. Note that the gradually decreasing function F(x) may be constant.

Therefore, the value of the dosage probability on each date of the dosage period, which is determined according to Expression (1), is gradually lowered from the dosage start date to the dosage end date according to the prescription amount N or the gradually decreasing function F(x). Note that the dosage probability for the pharmaceutical in the period other than the dosage period is set to "0." In the case where the gradually decreasing function F(x) is set to a constant, the dosage probability in the dosage period for the processing-target pharmaceutical takes a constant value.

In Step S48, the determiner 52 sets a pharmaceutical that has not yet been set as the processing target out of the pharmaceuticals (prescription medicines) the information of which has been extracted from the pharmaceutical receipt information, as the processing target. In other words, a pharmaceutical having a dosage form code of any of "2," "4," and "9" is set as the processing-target pharmaceutical.

In Step S49, by using the dosage period Ta for the first processing-target pharmaceuticals, the dosage schedule generator 53 determines the dosage period for the processing-target pharmaceutical and sets the dosage probability for the pharmaceutical to "1."

For example, the dosage schedule generator 53 sets the longest dosage period Ta among the dosage periods Ta for the first processing-target pharmaceuticals, as the dosage period for the processing-target pharmaceutical as it is. Further, the dosage schedule generator 53 sets the dosage probability in the dosage period for the processing-target pharmaceutical to "1" and the dosage probability in other periods to "0."

As the dosage period and the dosage probability for the pharmaceutical in a dosage form of the oral liquid medication, the injection, or the material, the dosage period and the dosage probability for the first processing-target pharmaceuticals is used as it is as described above. This is because the number of pharmaceuticals in the dosage form of the oral liquid medication, the injection, or the material is statistically small. The survey of the applicant of the subject application, which is based on the number of prescription cases for each dosage form, shows that the number of cases with the oral medication, the topical medication, and the one-shot medicine is about 99.5% of all the cases.

In Step S50, the dosage schedule generator 53 sets the information indicating the dosage period and the dosage probability for each pharmaceutical that are determined in the processing of Steps S43, S45, S47, and S49, as the dosage schedule information. The dosage schedule generation processing is terminated.

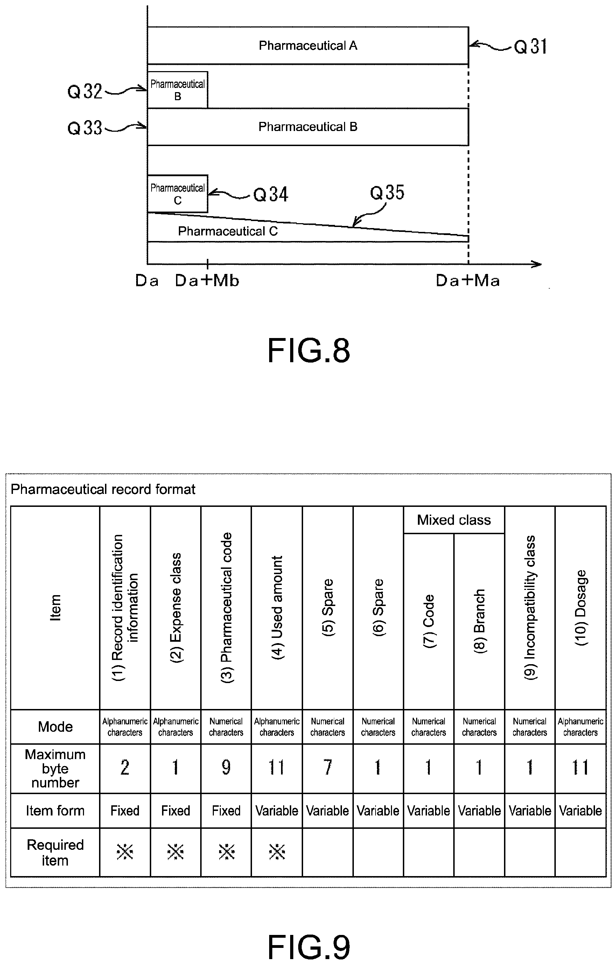

With this, for example, the dosage schedule information shown in Fig. 8 is obtained. Note that a horizontal direction in the figure indicates a date and a vertical direction in the figure indicates a dosage probability for a pharmaceutical.

The example shown in Fig. 8 is an example in which information on a pharmaceutical A being an oral medication, a pharmaceutical B being a topical medication, and a pharmaceutical C being a one-shot medicine are extracted from the pharmaceutical receipt information. Here, it is assumed that a dispensing date of the pharmaceutical A, the pharmaceutical B, and the pharmaceutical C is Da, the number of prescription days of the pharmaceutical A is Ma=14 days, and the number of prescription days of the pharmaceutical B and the pharmaceutical C is Mb=1 day. It should be noted that it is assumed that the number of actually required dosing days of the pharmaceutical B and the pharmaceutical C is equal to that of the pharmaceutical A, i.e., Ma=14 days.

In Fig. 8, a rectangle shown by the arrow Q31 indicates a dosage schedule and a dosage probability for the pharmaceutical A. In this example, a vertical height of the rectangle shown by the arrow Q31 indicates a value of the dosage probability. In the dosage schedule of the pharmaceutical A shown by the arrow Q31, the dispensing date Da is set as a dosage start date, (Da+Ma-1) is set as a dosage end date, and the dosage probability on each date in the dosage period is set to one.

Further, a rectangle shown by the arrow Q32 indicates a dosage schedule of the pharmaceutical B determined based on a dispensing date and a dispensing quantity (number of prescription days) of the pharmaceutical B. In the dosage schedule of the pharmaceutical B shown by the arrow Q32, the dosage period is determined based on the number of prescription days Mb of the pharmaceutical B, and hence the number of dosing days is smaller than the number of actually required days.

In contrast, by determining the dosage schedule of the pharmaceutical B by the dosage schedule generation processing described with reference to Fig. 7, a dosage schedule shown by the arrow Q33 is obtained as the dosage schedule of the pharmaceutical B. Note that a vertical height of the rectangle of the arrow Q33 indicates a value of the dosage probability.

In the dosage schedule shown by the arrow Q33, the dosage schedule of the pharmaceutical B is the same as the dosage schedule of the pharmaceutical A and the dosage probability in the dosage period takes a constant value.

In addition, the rectangle shown by the arrow Q34 indicates a dosage schedule of the pharmaceutical C determined based on a dispensing date and a dispensing quantity (number of prescription days) of the pharmaceutical C. The dosage schedule of the pharmaceutical C shown by the arrow Q34 is shorter than an actually required dosage period as in the pharmaceutical B.

In contrast, by determining the dosage schedule of the pharmaceutical C by the dosage schedule generation processing described with reference to Fig. 7, a dosage schedule shown by the arrow Q35 is obtained as the dosage schedule of the pharmaceutical C. Note that a vertical height of the figure shown by the arrow Q35 indicates a value of the dosage probability.

In the dosage schedule shown by the arrow Q35, the dosage schedule of the pharmaceutical C is the same as the dosage schedule of the pharmaceutical A. The dosage probability in the dosage period becomes linearly lower from the dosage start date to the dosage end date. In other words, the dosage probability is gradually lowered.

In the above-mentioned manner, the data center 14 determines the dosage period Ta for the first processing-target pharmaceuticals, calculates the dosage period for the other pharmaceuticals by using the dosage period Ta, and also calculates the dosage probability for each pharmaceutical. In this manner, both of the dosage schedule and the dosage probability are determined, and hence it becomes possible to obtain a wider variety of information on the dosage by using the obtained dosage schedule information. For example, with the dosage probability, it is possible to obtain a probability of co-administration occurrence of pharmaceuticals.

<Third Embodiment>

<Estimation Equation for Number of Dosing Days>

In the above, the dosage period for the other pharmaceuticals are determined using the dosage period for the first processing-target pharmaceuticals. However, in such processing, if the pharmaceutical receipt information does not include the information on the first processing-target pharmaceuticals, it is impossible to determine the dosage period for the other pharmaceuticals.

For example, in the case where the pharmaceutical receipt information includes information on an oral medication that is the first processing-target pharmaceutical and a topical medication that is not the first processing-target pharmaceutical, it is possible to estimate a dosage period for the topical medication. However, in the case where the pharmaceutical receipt information includes only the information on the topical medication, it is impossible to estimate an accurate dosage period for the topical medication.

Therefore, by utilizing the fact that the receipt has much information, a correlation between a used amount of a pharmaceutical that is not the first processing target such as a topical medication and a dosage period for the first processing-target pharmaceutical such as an oral medication may be used to prepare an estimation equation in advance and estimate a dosage period for the pharmaceutical that is not the first processing target according to the estimation equation. Hereinafter, a case where the estimation equation is determined by statistical processing based on the dosage period Ta of the oral medication and the used amount of the topical medication prescribed on the same date as the oral medication will be described as an example.

Regarding prescription of the topical medication, a single tube of a medicine for a plurality of days is often prescribed, for example. In other words, prescription granularity is often larger than that of the oral medication. Therefore, those taking large values out of the used amounts of the topical medications and the dosage periods Ta of the oral medications are used in the statistical processing.

This is because, if the dosage period Ta for the oral medication prescribed on the same date as the topical medication is short, that is, the actually required dosage period for the topical medication is short, the prescribed topical medication more than necessary is often prescribed. In other words, as the dosage period Ta becomes longer, the inconsistency between the prescription amount of the topical medication that will be used in the same period and the actually required topical medication amount becomes smaller.

In calculation of the estimation equation, a used amount of a particular topical medication for which the estimation equation is to be obtained and a dosage period Ta of an arbitrary oral medication prescribed on the same date as the topical medication are prepared.

For example, a used amount in a pharmaceutical record of the pharmaceutical receipt information as shown in Fig. 9 only needs to be used for data of the used amount of the topical medication. The used amount in the pharmaceutical record indicates a used amount of a prescribed pharmaceutical. For example, if the used amount is three (tablets), "3" is described in the field of the used amount.

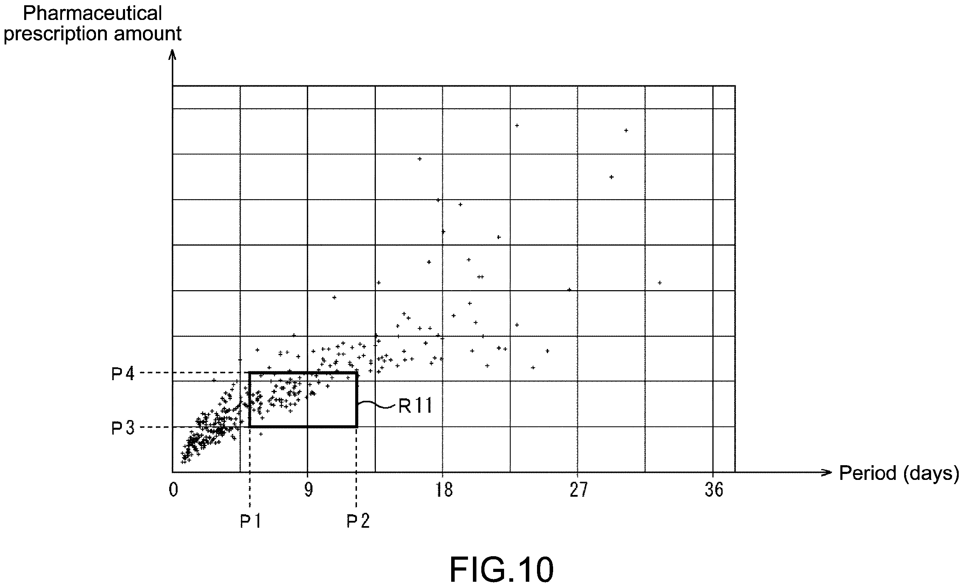

Data of corresponding combinations of used amounts of topical medications and dosage periods Ta of oral medications are prepared in this manner, such that a distribution shown in Fig. 10 is, for example, obtained as a distribution of this data. Note that, in Fig. 10, a vertical axis indicates the used amount of the topical medication, that is, the prescription amount and a horizontal axis indicates the number of days of the dosage period Ta of the oral medication.

In calculation of the estimation equation, only data of the data pieces, which is in a range defined by quartiles, is used. For example, a point P1 and a point P2 on a dosage period axis indicate a middle value and a third quartile of the number of days of the dosage period Ta, respectively. Further, a point P3 and a point P4 on a prescription amount axis indicate a middle value and a third quartile of the used amount of the topical medication, respectively.

By using only data in a region R11 between the middle value and the third quartile, a suitable consumption per day of the topical medication is estimated by a regression analysis of the prescription amount (used amount) and the dosage period Ta. The estimation equation can be obtained based on this consumption. That is, the estimation equation is calculated by excluding outliers of the prepared data and using only data larger than the average value.

When the prescription amount of the topical medication as the processing target, that is, the dispensing quantity is substituted, the thus obtained estimation equation is a function for obtaining an accurate dosage period for the topical medication.

Data of all the oral medications prescribed on the same date as the topical medication are basically used for calculation of the estimation equation. However, if there is much data available and data on the ages, the sexes, and the like of patients is also available, the estimation equation may be determined for each age or sex by using data of the same age or sex.

In contrast, if there is not much data available, in order to obtain sufficient data pieces for calculating an estimation equation, data pieces may be added for each pharmacotherapeutic classification to which the topical medication as the processing target belong. In other words, not only the data of the processing-target pharmaceuticals but also data of similar pharmaceuticals may also be used for calculating the estimation equation. In this case, for example, the estimation equation is obtained for each pharmacotherapeutic classification. Alternatively, data pieces may be added for each pharmaceutical classified according to the NHI (National Health Insurance) price listing code that is called YJ code and the added data pieces may be used for determining the estimation equation.

<Configuration Example of Data Center>

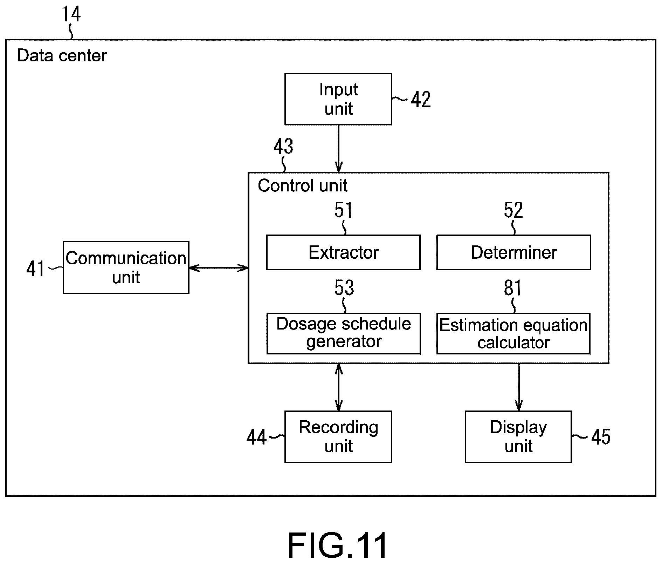

In the case where the estimation equation is, as described above, determined based on the information on the prescription medicines such as the pharmaceutical receipt information and the estimation equation is used to calculate the dosage period for the topical medication, the data center 14 is configured as shown in, for example, Fig. 11. Note that, in Fig. 11, portions corresponding to those in Fig. 2 will be denoted by the same symbols and descriptions thereof will be omitted.

The data center 14 shown in Fig. 11 is constituted of a communication unit 41, an input unit 42, a control unit 43, a recording unit 44, and a display unit 45. The configuration of the data center 14 in Fig. 11 has a configuration different from that of the data center 14 shown in Fig. 2 in that the control unit 43 includes an estimation equation calculator 81 but has the same configurations in the other points.

In other words, the control unit 43 includes an extractor 51, a determiner 52, a dosage schedule generator 53, and the estimation equation calculator 81.

Appropriately based on information on the prescription medicines such as the pharmaceutical receipt information recorded in the recording unit 44, the estimation equation calculator 81 performs the statistical processing described with reference to Fig. 10 and calculates an estimation equation of each topical medication. Further, the estimation equation calculator 81 retains the calculated estimation equations.

Appropriately using the estimation equations retained by the estimation equation calculator 81, the dosage schedule generator 53 calculates a dosage period for the topical medication.

<Explanation of Dosage Schedule Generation Processing>

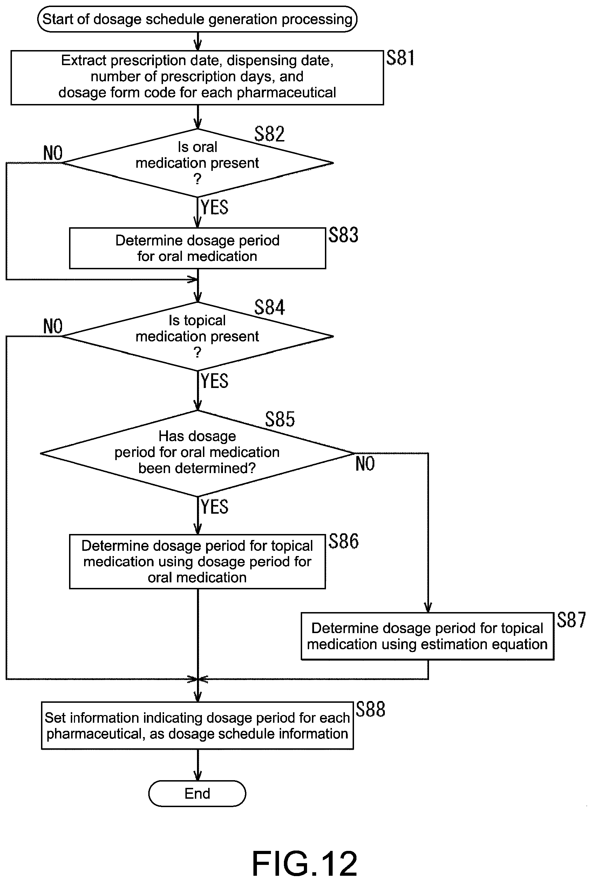

Next, referring to a flowchart of Fig. 12, the dosage schedule generation processing performed by the data center 14 shown in Fig. 11 will be described. Here, a case where the pharmaceuticals the information of which is extracted from the pharmaceutical receipt information are an oral medication and a topical medication and dosage schedule information of these pharmaceuticals is generated will be described.

In Step S81, the extractor 51 reads out the pharmaceutical receipt information from the recording unit 44 and performs analysis processing on the readout pharmaceutical receipt information, to thereby extract, from the pharmaceutical receipt information, a prescription date, a dispensing date, the number of prescription days, and a dosage form code of each prescription for each pharmaceutical. For example, a dispensing quantity is extracted as the number of prescription days.

In Step S82, the determiner 52 judges, based on a dosage form code of each pharmaceutical extracted from the pharmaceutical receipt information, whether or not an oral medication is present in the pharmaceuticals. In Step S82, if it is judged that the oral medication is not present, the processing proceeds to Step S84.

In contrast, if it is judged in Step S82 that the oral medication is present, then in Step S83, the dosage schedule generator 53 sets the oral medication the information of which has been extracted from the pharmaceutical receipt information, as a processing target, and determines a dosage period Ta for the oral medication based on the dispensing date and the number of prescription days of the oral medication. For example, in Step S83, the same processing as the processing of Step S13 of Fig. 5 is performed to determine the dosage period.

If the dosage period is determined in Step S83 or it is judged in Step S82 that the oral medication is not present, the processing of Step S84 is performed.

In other words, in Step S84, the determiner 52 judges, based on the dosage form code extracted from the pharmaceutical receipt information, whether or not a topical medication is present in the pharmaceuticals.

If it is judged in Step S84 that the topical medication is not present, the processing proceeds to Step S88.

On the other hand, if it is judged in Step S84 that the topical medication is present, in Step S85, the dosage schedule generator 53 judges whether or not the dosage period for the oral medication as the first processing-target pharmaceutical has been determined. For example, if the processing of Step S83 has been performed, it is judged that the dosage period for the oral medication has been determined.

If it is judged in Step S85 that the dosage period Ta for the oral medication has been determined, then in Step S86, the dosage schedule generator 53 determines a dosage period for the topical medication by using the determined dosage period Ta for the oral medication.

Specifically, with respect to each topical medication the information of which has been extracted from the pharmaceutical receipt information, the dosage period for the topical medication is determined by the same processing as the processing of Step S15 of Fig. 5. For example, the longest dosage period Ta among the dosage periods Ta for the oral medications is set as the dosage period for the topical medication as it is.

When the dosage period for the topical medication is determined by the processing of Step S86, then the processing proceeds to Step S88.

Further, if it is judged in Step S85 that the dosage period Ta for the oral medication has not been determined, in other words, if the pharmaceutical receipt information does not include the information on the oral medication, the processing proceeds to Step S87.

In Step S87, with respect to the topical medication the information of which has been extracted from the pharmaceutical receipt information, the dosage schedule generator 53 reads out the estimation equation of the topical medication from the estimation equation calculator 81 and substitutes the number of prescription days (dispensing quantity) of the topical medication in the estimation equation, to thereby determine the dosage period for the topical medication.

It is judged in Step S85 that the dosage period Ta for the oral medication has not been determined in the case where the dispensing date of the oral medication is not within a particular period including the dispensing date of the topical medication, i.e., a period set as a range for creating the receipt. In other words, it is the case where the information on the oral medication is not included in the same receipt.

Even in such a case, the dosage schedule generator 53 can determine the dosage period for the topical medication set as the processing target by using the estimation equation prepared in advance without using the dosage period Ta for the oral medication.

When the dosage period for the topical medication is determined by the processing of Step S87, then the processing proceeds to Step S88.

If it is judged in Step S84 that the topical medication is not present or the dosage period for the topical medications is determined in Step S86 or Step S87, the processing of Step S88 is performed. In Step S88, the dosage schedule generator 53 sets information indicating the dosage period for the pharmaceutical, i.e., the oral medication or the topical medication which is determined in the above-mentioned processing, as the dosage schedule information. The dosage schedule generation processing is terminated.

The dosage schedule information generated by the dosage schedule generator 53 is transmitted by the communication unit 41 to the portable terminal apparatus 11 and utilized for various application programs.

In the above-mentioned manner, if the dosage period Ta for the oral medication has been determined, the data center 14 determines the dosage period for the topical medication by using the dosage period Ta. If the dosage period Ta for the oral medication has not been determined, the data center 14 determines the dosage period for the topical medication by using the estimation equation.

By using the estimation equation depending on needs in the above-mentioned manner, even if the pharmaceutical receipt information does not include the information on the oral medication, it is possible to obtain more accurate dosage schedule information on the topical medication. In other words, it is possible to obtain more accurate information on the dosage.

Although the oral medication and the topical medication have been exemplified, the dosage period for the first processing-target pharmaceutical described above and the estimation equation determined based on the used amount of any other pharmaceuticals that are not the first processing targets may be used for determining the dosage period for the other pharmaceuticals.

By the way, the above-mentioned series of processing may be executed by hardware or may be executed by software. When the series of processing executing are executed by software, a program configuring the software is installed into the computer. Here, the computer includes, for example, a computer incorporated in dedicated hardware and a general-purpose personal computer capable of executing various functions by installing various programs.

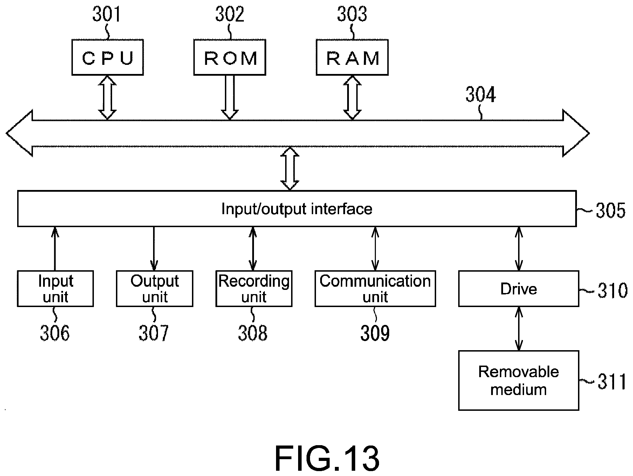

Fig. 13 is a block diagram showing a configuration example of hardware of a computer that executes the above-mentioned series of processing according to a program.

In the computer, a CPU (Central Processing Unit) 301, a ROM (Read Only Memory) 302, a RAM (Random Access Memory) 303 are connected to one another via a bus 304.

To the bus 304, further connected is an input/output interface 305. To the input/output interface 305, connected are an input unit 306, an output unit 307, a recording unit 308, a communication unit 309, and a drive 310.

The input unit 306 is, for example, a keyboard, a mouse, a microphone, or an imaging element. The output unit 307 is, for example, a display or a speaker. The recording unit 308 is, for example, a hard disk or a non-volatile memory. The communication unit 309 is, for example, a network interface. The drive 310 drives a removable medium 311 such as a magnetic disk, an optical disc, an magneto-optic disk, and a semiconductor memory.

In the thus configured computer, by the CPU 301 loading, for example, a program recorded in the recording unit 308 into the RAM 303 via the input/output interface 305 and the bus 304, the above-mentioned series of processing are performed.

A program executed by the computer (CPU 301) can be recorded and provided in/as the removable medium 311 as, for example, a package medium. Further, the program can be provided via a wired or wireless transmission medium such as a local area network, the Internet, and digital satellite broadcasting.

In the computer, the program can be installed into the recording unit 308 via the input/output interface 305 by mounting the removable medium 311 on the drive 310. Further, the program can be received by the communication unit 309 and installed into the recording unit 308 via the wired or wireless transmission medium. Otherwise, the program can be installed into the ROM 302 or the recording unit 308 in advance.

Note that the program executed by the computer may be a program for performing processing in a time sequence in the order described herein or may be a program for performing processing in parallel or at a necessary timing, for example, when called.

Further, the embodiments of the present technology are not limited to the above-mentioned embodiments and various modifications can be made without departing from the gist of the present technology.

For example, the present technology can take a cloud computing configuration in which a single function is shared with a plurality of apparatuses via a network and cooperatively processed.

Further, the steps described in the above-mentioned flowcharts can be executed by a single apparatus and can also be shared with a plurality of apparatuses and executed.

In addition, in the case where a single step include a plurality of processes, the plurality of processes of the single step can be executed by a single apparatus and can also be shared with a plurality of apparatuses and executed.

In addition, the present technology may also take the following configurations.

[1] An information processing apparatus, including:

a determiner that identifies, based on information on pharmaceuticals prescribed for a user, dosage forms of the pharmaceuticals; and
a dosage schedule generator that sets the pharmaceutical in a predetermined particular dosage form as a first processing-target pharmaceutical and determines, based on the dosage period for the first processing-target pharmaceutical, a dosage period for the pharmaceutical in another dosage form different from the particular dosage form, the pharmaceutical in the other dosage form having a dispensing date within a particular period including a dispensing date of the first processing-target pharmaceutical.

[2] The information processing apparatus according to [1], in which
the dosage schedule generator determines, based on the dispensing dates and the number of prescription days of the pharmaceuticals included in information on the pharmaceuticals, the dosage period for the first processing-target pharmaceutical.
[3] The information processing apparatus according to [1] or [2], in which
the dosage schedule generator sets the number of days of the dosage period for the first processing-target pharmaceutical as the number of days of the dosage period for the pharmaceutical in the other dosage form.
[4] The information processing apparatus according to any one of [1] to [3], in which
the dosage schedule generator sets the longest dosage period among the dosage periods for the plurality of first processing-target pharmaceuticals, as the dosage period for the pharmaceutical in the other dosage form.
[5] The information processing apparatus according to any one of [1] to [4], in which
the particular dosage form is an oral medication, an infusion, or a decoction.
[6] The information processing apparatus according to any one of [1] to [5], in which
the other dosage form is an oral liquid medication, a one-shot medicine, an injection, a topical medication, or a material.
[7] The information processing apparatus according to any one of [1] to [6], in which
the dosage schedule generator generates information indicating the dosage period for the pharmaceuticals and a dosage probability for the pharmaceuticals, as dosage schedule information.
[8] The information processing apparatus according to [7], in which
the dosage schedule generator calculates, based on the dosage period, the dosage probability for the pharmaceutical in a dosage form of a one-shot medicine such that the dosage probability on each date is gradually lowered according to the number of days from a prescription date.

[9] The information processing apparatus according to any one of [1] to [8], in which the dosage schedule generator calculates, if the dispensing date of the first processing-target pharmaceutical is not present in the particular period including the dispensing date of the pharmaceutical in the other dosage form, the dosage period for the pharmaceutical in the other dosage form by using an estimation equation calculated in advance by statistical processing based on the dosage period for the first processing-target pharmaceutical and a used amount of the pharmaceutical in the other dosage form.

Description of Reference Numerals

11    portable terminal apparatus
14    data center
41    communication unit
44    recording unit
51    extractor
52    determiner
53    number-of-dosing-days calculator
81    estimation equation calculator

**Claims**

1. An information processing apparatus, comprising:

   a determiner (52) that is adapted to identify, based on information on pharmaceuticals prescribed for a user, dosage forms of the pharmaceuticals; and
   a dosage schedule generator (53) that is adapted to set a first pharmaceutical of the pharmaceuticals in a predetermined particular dosage form of the dosage forms as a first processing-target pharmaceutical having a first dosage period based on the information on the pharmaceuticals and determine, based on the first dosage period for the first processing-target pharmaceutical, a second dosage period for a second pharmaceutical of the pharmaceuticals in another dosage form of the dosage forms different from the particular dosage form, wherein
   the dosage schedule generator is adapted to calculate, if the dispensing date of the first processing-target pharmaceutical is not present in the particular period including the dispensing date of the second pharmaceutical in the another dosage form, the second dosage period for the second pharmaceutical in the another dosage form by using an estimation equation calculated in advance by statistical processing based on the first dosage period for the first processing-target pharmaceutical and a used amount of the second pharmaceutical in the another dosage form.

2. The information processing apparatus according to claim 1, wherein the particular dosage form is an oral medication, an infusion, or a decoction.

3. The information processing apparatus according to any preceding claim, wherein in calculation of the estimation equation, only data on the first dosage period between a middle value and a third quartile of a number of days of the dosage first period are used.

4. The information processing apparatus according to any preceding claim, wherein in calculation of the estimation equation, only data on the used amount of the second pharmaceutical in the another dosage form between a middle value and a third quartile of the used amount are used.

5. The information processing apparatus according to any preceding claim, wherein in calculation of the estimation equation a regression analysis of the used amount and the first dosage period is used.

6. The information processing apparatus according to any preceding claim, wherein the estimation equation is further determined on a patient age basis or on a patient sex basis.

7.  The information processing apparatus according to any of claims 1-5, wherein
    the estimation equation is determined using data for a pharmacotherapeutic classification to which the first processing-target pharmaceutical belongs.

8.  The information processing apparatus according to claim 1 or claim 2, wherein
    the another dosage form is an oral liquid medication, a one-shot medicine, an injection, a topical medication, or a material.

9.  An information processing method, comprising the steps of:

    identifying, based on information on pharmaceuticals prescribed for a user, dosage forms of the pharmaceuticals; and
    setting a first pharmaceutical of the pharmaceuticals in a predetermined particular dosage form of the dosage forms as a first processing-target pharmaceutical having a first dosage period based on the information on the pharmaceuticals and determining, based on the first dosage period for the first processing-target pharmaceutical, a second dosage period for a second pharmaceutical of the pharmaceuticals in another dosage form of the dosage forms different from the particular dosage form,
    wherein, if the dispensing date of the first processing-target pharmaceutical is not present in the particular period including the dispensing date of the second pharmaceutical in the another dosage form, the second dosage period for the second pharmaceutical in the another dosage form is calculated by using an estimation equation calculated in advance by statistical processing based on the first dosage period for the first processing-target pharmaceutical and a used amount of the second pharmaceutical in the another dosage form.

10. A program that causes a computer to execute processing comprising the steps of claim 9.


**Patentansprüche**

1.  Informationsverarbeitungsvorrichtung, umfassend:

    eine Bestimmungseinrichtung (52), die eingerichtet ist zum Identifizieren von Dosierungsformen von Arzneimitteln basierend auf Information zu einem Benutzer verschriebenen Arzneimitteln; und
    einen Dosierungsplanerzeuger (53), der eingerichtet ist zum Vorgeben eines ersten Arzneimittels der Arzneimittel in einer vorbestimmten besonderen Dosierungsform der Dosierungsformen als ein erstes Verarbeitungszielarzneimittel, aufweisend einen ersten Dosierungszeitraum, basierend auf der Information zu den Arzneimitteln, und zum Festlegen eines zweiten Dosierungszeitraums für ein zweites Arzneimittel der Arzneimittel in einer anderen Dosierungsform der Dosierungsformen, die sich von der besonderen Dosierungsform unterscheidet, basierend auf dem ersten Dosierungszeitraum für das erste Verarbeitungszielarzneimittel, wobei
    der Dosierungsplanerzeuger, falls das Verabreichungsdatum des ersten Verarbeitungszielarzneimittels in dem besonderen Zeitraum, der das Verabreichungsdatum des zweiten Arzneimittels in der anderen Dosierungsform aufweist, nicht vorliegt, eingerichtet ist zum Berechnen des zweiten Dosierungszeitraums für das zweite Arzneimittel in der anderen Dosierungsform unter Verwendung einer Schätzungsgleichung, im Voraus berechnet mittels statistischer Verarbeitung basierend auf dem ersten Dosierungszeitraum für das erste Verarbeitungszielarzneimittel und einer Verbrauchsmenge des zweiten Arzneimittels in der anderen Dosierungsform.

2.  Informationsverarbeitungsvorrichtung nach Anspruch 1, wobei
    die besondere Dosierungsform eine orale Medikation, eine Infusion oder ein Dekokt ist.

3.  Informationsverarbeitungsvorrichtung nach einem der vorstehenden Ansprüche, wobei
    bei der Berechnung der Schätzungsgleichung nur Daten zu dem ersten Dosierungszeitraum zwischen einem Mittelwert und einem dritten Quartil einer Anzahl von Tagen des ersten Dosierungszeitraums verwendet werden.

4.  Informationsverarbeitungsvorrichtung nach einem der vorstehenden Ansprüche, wobei
    bei der Berechnung der Schätzungsgleichung nur Daten zu der Verbrauchsmenge des zweiten Arzneimittels in der anderen Dosierungsform zwischen einem Mittelwert und einem dritten Quartil der Verbrauchsmenge verwendet werden.

5.  Informationsverarbeitungsvorrichtung nach einem der vorstehenden Ansprüche, wobei

bei der Berechnung der Schätzungsgleichung eine Regressionsanalyse der Verbrauchsmenge und des ersten Dosierungszeitraums verwendet wird.

6. Informationsverarbeitungsvorrichtung nach einem der vorstehenden Ansprüche, wobei die Schätzungsgleichung ferner auf der Grundlage eines Alters der Patienten oder auf der Grundlage eines Geschlechts der Patienten ermittelt wird.

7. Informationsverarbeitungsvorrichtung nach einem der Ansprüche 1-5, wobei die Schätzungsgleichung unter Verwendung von Daten für eine pharmakotherapeutische Klassifizierung ermittelt wird, zu der das erste Verarbeitungszielarzneimittel gehört.

8. Informationsverarbeitungsvorrichtung nach Anspruch 1 oder Anspruch 2, wobei die andere Dosierungsform eine flüssige orale Medikation, ein einmal verabreichtes Arzneimittel, eine Injektion, ein topisches Arzneimittel oder ein Material ist.

9. Informationsverarbeitungsverfahren, umfassend die Schritte:

Identifizieren von Dosierungsformen der Arzneimittel basierend auf Information zu einem Benutzer verschriebenen Arzneimitteln; und

Vorgeben eines ersten Arzneimittels der Arzneimittel in einer vorbestimmten besonderen Dosierungsform der Dosierungsformen als ein erstes Verarbeitungszielarzneimittel, aufweisend einen ersten Dosierungszeitraum, basierend auf der Information zu den Arzneimitteln, und Festlegen eines zweiten Dosierungszeitraums für ein zweites Arzneimittel der Arzneimittel in einer anderen Dosierungsform der Dosierungsformen, die sich von der besonderen Dosierungsform unterscheidet, basierend auf dem ersten Dosierungszeitraum für das erste Verarbeitungszielarzneimittel,

wobei, falls das Verabreichungsdatum des ersten Verarbeitungszielarzneimittels in dem besonderen Zeitraum, der das Verabreichungsdatum des zweiten Arzneimittels in der anderen Dosierungsform aufweist, nicht vorliegt, der zweite Dosierungszeitraum für das zweite Arzneimittel in der anderen Dosierungsform berechnet wird unter Verwendung einer Schätzungsgleichung, im Voraus berechnet mittels statistischer Verarbeitung basierend auf dem ersten Dosierungszeitraum für das erste Verarbeitungszielarzneimittel und einer Verbrauchsmenge des zweiten Arzneimittels in der anderen Dosierungsform.

10. Programm, das einen Rechner veranlasst, eine die Schritte nach Anspruch 9 umfassende Verarbeitung durchzuführen.

**Revendications**

1. Appareil de traitement d'informations, comprenant :

une unité de détermination (52) qui est capable d'identifier, d'après des informations concernant des produits pharmaceutiques prescrits à un utilisateur, des formes de dosage des produits pharmaceutiques ; et

un générateur de posologie (53) qui est capable de définir un premier produit pharmaceutique des produits pharmaceutiques dans une forme de dosage particulière prédéterminée des formes de dosage en tant que premier produit pharmaceutique cible de traitement ayant une première période de dosage basée sur les informations concernant les produits pharmaceutiques et de déterminer, d'après la première période de dosage du premier produit pharmaceutique cible de traitement, une deuxième période de dosage pour un deuxième produit pharmaceutique des produits pharmaceutiques dans une autre forme de dosage des formes de dosage, différente de la forme de dosage particulière, dans lequel

le générateur de posologie est capable de calculer, si la date d'administration du premier produit pharmaceutique cible de traitement n'est pas présente dans la période particulière contenant la date d'administration du deuxième produit pharmaceutique dans l'autre forme de dosage, la deuxième période de dosage du deuxième produit pharmaceutique dans l'autre forme de dosage au moyen d'une équation d'estimation calculée à l'avance par un traitement statistique basé sur la première période de dosage du premier produit pharmaceutique cible de traitement et une quantité utilisée du deuxième produit pharmaceutique dans l'autre forme de dosage.

2. Appareil de traitement d'informations selon la revendication 1, dans lequel la forme de dosage particulière est une médication orale, une infusion ou une décoction.

3. Appareil de traitement d'informations selon l'une quelconque des revendications précédentes, dans lequel dans le calcul de l'équation d'estimation, seules sont utilisées les données concernant la première période de dosage entre une valeur médiane et un troisième quartile d'un nombre de jours de la première période de dosage.

4. Appareil de traitement d'informations selon l'une quelconque des revendications précédentes, dans lequel dans le calcul de l'équation d'estimation, seules sont utilisées les données concernant la quantité utilisée du deuxième produit pharmaceutique dans l'autre forme de dosage entre une valeur médiane et un troisième quartile de la quantité utilisée.

5. Appareil de traitement d'informations selon l'une quelconque des revendications précédentes, dans lequel dans le calcul de l'équation d'estimation, une analyse de régression de la quantité utilisée et de la première période de dosage est utilisée.

6. Appareil de traitement d'informations selon l'une quelconque des revendications précédentes, dans lequel l'équation d'estimation est également déterminée en fonction de l'âge du patient ou en fonction du sexe du patient.

7. Appareil de traitement d'informations selon l'une quelconque des revendications 1 à 5, dans lequel l'équation d'estimation est déterminée à l'aide de données concernant une classification de pharmacothérapie à laquelle le premier produit pharmaceutique cible de traitement appartient.

8. Appareil de traitement d'informations selon la revendication 1 ou la revendication 2, dans lequel l'autre forme de dosage est une médication liquide orale, un médicament à administration unique, une injection, une médication topique ou une matière.

9. Procédé de traitement d'informations, comprenant les étapes consistant à :

identifier, d'après des informations concernant des produits pharmaceutiques prescrits à un utilisateur, des formes de dosage des produits pharmaceutiques ; et
définir un premier produit pharmaceutique des produits pharmaceutiques dans une forme de dosage particulière prédéterminée des formes de dosage en tant que premier produit pharmaceutique cible de traitement ayant une première période de dosage basée sur les informations concernant les produits pharmaceutiques et déterminer, d'après la première période de dosage du premier produit pharmaceutique cible de traitement, une deuxième période de dosage pour un deuxième produit pharmaceutique des produits pharmaceutiques dans une autre forme de dosage des formes de dosage, différente de la forme de dosage particulière,
dans lequel, si la date d'administration du premier produit pharmaceutique cible de traitement n'est pas présente dans la période particulière contenant la date d'administration du deuxième produit pharmaceutique dans l'autre forme de dosage, la deuxième période de dosage du deuxième produit pharmaceutique dans l'autre forme de dosage est calculée au moyen d'une équation d'estimation calculée à l'avance par un traitement statistique basé sur la première période de dosage du premier produit pharmaceutique cible de traitement et une quantité utilisée du deuxième produit pharmaceutique dans l'autre forme de dosage.

10. Programme qui amène un ordinateur à exécuter un traitement comprenant les étapes de la revendication 9.

FIG.1

**FIG.2**

Prescription basic record format

Q11

| Item | (1) Record identification information | (2) No. | (3) Dosage form code | Usage | | Medication expense per unit | | | | |
|---|---|---|---|---|---|---|---|---|---|---|
| | | | | (4) Usage code | (5) Special instruction | (6) Total | (7) First public expense | (8) Second public expense | (9) Third public expense | (10) Fourth public expense |
| Mode | Alphanumeric characters | Numerical characters | Numerical characters | Numerical characters | Kanji characters | Numerical characters | Numerical characters | Numerical characters | Numerical characters | Numerical characters |
| Maximum byte number | 2 | 2 | 1 | 3 | 80 | 7 | 7 | 7 | 7 | 7 |
| Item form | Fixed | Fixed | Fixed | Variable | Variable | Variable | Variable | Variable | Variable | Variable |
| Required item | ※ | ※ | ※ | | | ※ | | | | |

Q12

| Code name | Code | Contents |
|---|---|---|
| Dosage form code | 1 | Oral medication |
| | 2 | Oral liquid medication |
| | 3 | One-shot medicine |
| | 4 | Injection |
| | 5 | Topical medication |
| | 6 | Infusion |
| | 7 | Decoction |
| | 9 | Material |

FIG.3

Dispensing information record format

| Item | (1) Record identification information | (2) Doctor number | (3) Prescription date | (4) Dispensing date | (5) Number of receiving prescriptions | (6) Dispensing quantity | Dispensing fee | | |
|---|---|---|---|---|---|---|---|---|---|
| | | | | | | | (7) Expense class | (8) Calculation class | (9) Calculation destination No. |
| Mode | Alphanumeric characters | Numerical characters | Numerical characters | Numerical characters | Numerical characters | Numerical characters | Alphanumeric characters | Numerical characters | Numerical characters |
| Maximum byte number | 2 | 2 | 7 | 7 | 2 | 3 | 1 | 1 | 2 |
| Item form | Fixed | Variable | Fixed | Fixed | Variable | Variable | Fixed | Fixed | Fixed |
| Required item | ※ | ※ | ※ | ※ | ※ | ※ | ※ | ※ | ※ |

# FIG.4

Start of dosage schedule generation processing

Extract prescription date, dispensing date, number of prescription days, and dosage form code for each pharmaceutical — S11

Set oral medication, infusion, and decoction as processing targets — S12

Determine dosage period for processing-target pharmaceuticals — S13

Set pharmaceuticals other than oral medication, infusion, and decoction as processing targets — S14

Determine dosage period for processing-target pharmaceuticals using dosage period for other pharmaceuticals — S15

Set information indicating dosage period for each pharmaceutical as dosage schedule information — S16

End

# FIG.5

| Medical treatment identification code (medical) | | | |
|---|---|---|---|
| Code name | Code | Inpatient medical care | Outpatient medical care |
| | 01 | Entire identification code | |
| | 11 | First visit | |
| | 12 | | Revisit |
| | 13 | Medical supervision and management | |
| | 14 | Home medical care | |
| | 21 | Oral medication | |
| | 22 | One-shot medicine | |
| | 23 | Topical medication | |
| | 24 | Dispensing | |
| | 25 | | Prescribing |
| | 26 | Narcotic and poisonous drug | |
| | 27 | Basic dispensing fee | |
| Medical treatment identification code | 28 | Others | |

(Code 21–28: Medication)

FIG.6

Start of dosage schedule generation processing

Extract prescription date, dispensing date, number of
prescription days, and dosage form code for each pharmaceutical | S41

Set oral medication, infusion, and decoction as processing targets | S42

Determine dosage period and dosage probability
for processing-target pharmaceuticals | S43

Set topical medication as processing target | S44

Determine dosage period for processing-target pharmaceutical
using dosage period for other pharmaceuticals,
and set dosage probability to constant value | S45

Set one-shot medicine as processing target | S46

Determine dosage period for processing-target pharmaceutical
using dosage period for other pharmaceuticals, and set dosage
probability to constant value or gradually decreasing value | S47

Set pharmaceutical that has not yet been set
as processing target, as processing target | S48

Determine dosage period for processing-target pharmaceutical
using dosage period for other pharmaceuticals,
and set dosage probability to one | S49

Set information indicating dosage period and dosage probability
for each pharmaceutical as dosage schedule information | S50

End

FIG.7

Pharmaceutical A — Q31

Q32 — Pharmaceutical B

Q33 — Pharmaceutical B

Pharmaceutical C — Q34          Q35

Pharmaceutical C

$D a$   $D a + M b$          $D a + M a$

## FIG.8

Pharmaceutical record format

| Item | (1) Record identification information | (2) Expense class | (3) Pharmaceutical code | (4) Used amount | (5) Spare | (6) Spare | Mixed class | | (9) Incompatibility class | (10) Dosage |
| | | | | | | | (7) Code | (8) Branch | | |
|---|---|---|---|---|---|---|---|---|---|---|
| Mode | Alphanumeric characters | Alphanumeric characters | Numerical characters | Alphanumeric characters | Numerical characters | Numerical characters | Numerical characters | Numerical characters | Numerical characters | Alphanumeric characters |
| Maximum byte number | 2 | 1 | 9 | 11 | 7 | 1 | 1 | 1 | 1 | 11 |
| Item form | Fixed | Fixed | Fixed | Variable | Variable | Variable | Variable | Variable | Variable | Variable |
| Required item | ※ | ※ | ※ | ※ | | | | | | |

## FIG.9

FIG.10

FIG.11

Start of dosage schedule generation processing

Extract prescription date, dispensing date, number of prescription days, and dosage form code for each pharmaceutical — S81

S82 — Is oral medication present ?

NO

YES

Determine dosage period for oral medication — S83

S84 — Is topical medication present ?

NO

YES

S85 — Has dosage period for oral medication been determined?

NO

YES

Determine dosage period for topical medication using dosage period for oral medication — S86

Determine dosage period for topical medication using estimation equation — S87

Set information indicating dosage period for each pharmaceutical, as dosage schedule information — S88

End

FIG.12

FIG.13

EP 2 953 088 B1

**EP 2 953 088 B1**

**REFERENCES CITED IN THE DESCRIPTION**

**Patent documents cited in the description**

- JP 2004029985 A
- US 2006149480 A
- US 5758095 A